# NEUE EUROPÄISCHE PATENTSCHRIFT

(11) **EP 2 190 490 B2**
(45) Veröffentlichungstag und Bekanntmachung des Hinweises auf die Entscheidung über den Einspruch: **22.05.2019**
(45) Hinweis auf die Patenterteilung: 09.11.2011
(21) Anmeldenummer: 08832467.8
(22) Anmeldetag: 17.09.2008
(51) Int. Cl.: A61L 15/46, A61L 15/32

(54) **BIORESORBIERBARER GELATINEVLIESSTOFF**
BIORESORBABLE NONWOVEN FABRIC MADE OF GELATIN
NONTISSÉ BIODÉGRADABLE À BASE DE GÉLATINE

(30) Priorität: 18.09.2007 DE 102007044648
(43) Veröffentlichungstag der Anmeldung: 02.06.2010
(60) Teilanmeldung: 11006496.1
(73) Patentinhaber: Carl Freudenberg KG, 69469 Weinheim (DE)
(72) Erfinder: REIBEL, Denis, F-67850 Herrlisheim (FR); WALTER, Claudio, 64646 Heppenheim (DE); ALTMÜLLER, Bernd, 69488 Birkenau (DE)
(74) Vertreter: Banse & Steglich Patentanwälte PartmbB
(86) Internationale Anmeldenummer: PCT/EP2008/007757
(87) Internationale Veröffentlichungsnummer: WO 2009/036958

(56) Entgegenhaltungen:
- EP-A2- 0 624 665
- WO-A1-00/67694
- WO-A1-02/43743
- WO-A1-2004/002384
- WO-A1-2005/054553
- WO-A2-2007/122232
- CN-A- 1 270 240
- US-A1- 2004 001 880
- US-A1- 2004 110 439
- VERSUCHSPROTOKOLL, EINGEREICHT AM 22.10.2013, 2 SEITEN
- V.VICHAI ET AL: 'SULFORHODAMINE B COLORIMETRIC ASSAY FOR CYTOTOXICITY SCREENING' NATURE PROTOCOLS Bd. 1, Nr. 3, 01 August 2006, Seiten 1112 - 1116, XP055272660
- AN INTRODUCTION TO ELECTROSPINNING AND NANOFIBERS WORLD SCIENTIFIC PUBLISHING Nr. 6.1.2, 2005, Seiten 248 - 249
- MEGELSKI, S. ET AL: 'MICRO- AND NANOSTRUCTURED SURFACE MORPHOLOGY ON ELECTROSPUN POLYMER FIBERS' MACROMOLECULES Bd. 35, Nr. 22, Seiten 8456 - 8466, XP055073505

## Beschreibung

### Technisches Gebiet

Die Erfindung betrifft ein Rotationsspinnverfahren zur Herstellung eines Vliesstoffs.

### Stand der Technik

Derartige Vliesstoffe sind bereits aus dem Stand der Technik bekannt.

Insbesondere zeigt die WO 2004/002384 A1 einen Vliesstoff, welcher Silber aufweist und als Wundauflage verwendet wird. Als Faser-Material werden dort Polyurethane oder Polyacrylate vorgeschlagen.

Aus der US 200410910439 A1 ist ein Vliesstoff bekannt, der Gelatine aufweist und durch ein Elektrospinnverfahren gefertigt ist.

Vliesstoffe werden häufig für medizinische Anwendungen verwendet. Den als Rohmaterial für die Vliesstoffe vorliegenden Vliesen wird durch Wasserstrahiverfestigung, thermische oder chemische Verfestigung eine hinreichende Reißfestigkeit verliehen, damit diese als Verbandsmaterial eingesetzt werden können.

Die mechanischen oder chemischen Verfestigungsverfahren können jedoch die antimikrobielle oder antibiotische Ausrüstung der Fasern negativ beeinträchtigen, nämlich die wirksamen Stoffe in ihrer Wirkung hemmen oder sogar teilweise aus den Fasern herauslösen. Daher sind häufig aufwendige und teure Nachbehandlungsschritte notwendig, um die verfestigten Vliesstoffe in einen gebrauchstauglichen Zustand zu verbringen.

Des Weiteren ist nachteilig, dass Faserrohmaterial aus Polymeren, die eine ausreichende Verfestigung erlauben, häufig nicht wundverträglich, insbesondere nicht bioresorbierbar ist.

### Darstellung der Erfindung

Der Erfindung liegt daher die Aufgabe zugrunde, einen bioresorbierbaren Vliesstoff mit hinreichender Festigkeit kostengünstig zu fertigen.

Die vorliegende Erfindung löst die zuvor genannte Aufgabe durch die Merkmale des Patentanspruchs 1.

Erfindungsgemäß ist erkannt worden, dass Gelatine ein biologisch abbaubares Material darstellt, welches sich überraschend gut zu einem Vlies ablegen lässt. Des Weiteren wurde erkannt, dass die Fasern des Vlieses zum Teil ohne Phasengrenze ineinander übergehen, miteinander vernetzen und dadurch einen festen Verbund ausbilden. Der entstehende Vliesstoff zeigt ohne weitere Verfestigungsmaßnahmen überraschend eine hinreichend hohe Reißfestigkeit, um als Verbandsmaterial oder Wundauflage Verwendung zu finden. Der antimikrobiell wirksame Stoff und/ oder das Antibiotikum werden in ihrer Wirkung nicht durch Verfestigungsmaßnahmen negativ beeinträchtigt. Weiter ist erkannt worden, dass mittels eines Rotationsspinnverfahrens der Durchmesser der Fasern in einer engen Verteilung einstellbar ist. Durch das Rotationsspinnverfahren sind Fasern mit einem Durchmesser von im Mittel 0,3 bis 500 µm im Mittel 3 bis 200 µm und sogar im Mittel 5 bis 100 µm erzeugbar, die untereinander partiell durch die Gelatine vernetzt sind. Die enge Verteilung des Durchmessers der Fasern erlaubt einen homogenen und stabilen Aufbau des Vliesstoffs ohne teure zusätzliche Verfestigungsmaßnahmen.

Folglich ist die eingangs genannte Aufgabe gelöst.

Einige Fasern könnten miteinander verdrillt oder verschlungen sein oder eine verdrillte Struktur aufweisen. Die Vendrillungen oder Verschlingungen stellen sich überraschend während des Rotationsspinnens ein und begünstigen zusätzlich die Festigkeit und das Dehnungsverhalten des Vliesstoffs.

Einige Fasern könnten miteinander verschlungen sein und ein oder mehrere Faserbündel ausbilden. Durch die Verschlingungen einzelner Fasern werden diese zu Faserbündeln zusammengefasst und können gegeneinander reversibel verschoben werden. Hierdurch ist es möglich, den Vliesstoff zerstörungsfrei zu dehnen. Bei der Dehnung werden nämlich einzelne Fasern gezogen und relativ zu anderen Fasern verschoben. Durch die Verdrillungen oder Verschlingungen wird sogar begünstigt, dass die Fasern in ihre Position vor der Dehnung zurückkehren. Der Vliesstoff zeigt daher eine hohe Formstabilität.

Die Fasern sind ausschließlich aus Gelatine oder Derivaten der Gelatine gefertigt, wobei in den Fasern ein antimikrobieller Stoff und oder ein Antibiotikum vorliegt. Ein solcher Vliesstoff kann durch die Chemie des menschlichen Körpers abgebaut werden und ist daher nahezu vollständig bioresorbierbar. Dieser Vliesstoff kann in den menschlichen Körper eingebracht werden.

Der antimikrobiell wirksame Stoff und/ oder das Antibiotikum sind homogen in den Fasern verteilt. Hierdurch lässt sich eine allmähliche Abgabe des Stoffes und/oder des Antibiotikums mit langanhaltender Wirkung einstellen.

Der antimikrobiell wirksame Stoff und/ oder das Antibiotikum könnte in den Fasern nanoskalig vorliegen. Unter nanoskaligen Strukturen versteht man Bereiche jeglicher Morphologie, die zumindest in einer Raumrichtung Dimensionen im Nanometerbereich aufweisen. Hierdurch erlangt der antimikrobiell wirksame Stoff oder das Antibiotikum eine hohe Mobilität. Ein nanoskalig vorliegender antimikrobieller Stoff zeigt eine besonders hohe Reaktivität, wenn der Stoff mit Bakterien, Viren, Pilzen oder Sporen in Kontakt gebracht wird. Des Weiteren gibt der Vliesstoff den wirkenden Stoff sehr leicht an Medien ab, die mit diesem in Kontakt treten. Insoweit zeichnet sich der Vliesstoff durch eine hohe Abgabefähigkeit in Bezug auf den antimikrobiell wirkenden Stoff und/ oder das Antibiotikum aus.

Der antimikrobiell wirksame Stoff und/ oder das Antibiotikum könnte auf den Fasern verteilt sein. Hierdurch ist ein Besprühen des Vliesstoffs mit dem Stoff und/ oder dem Antibiotikum ermöglicht, um eine rasche Abgabe an den menschlichen Körper sicher zu stellen.

Der antimikrobiell wirksame Stoff könnte Silber enthalten. Silber ist besonders als antimikrobiell wirkender Stoff geeignet, da es für Menschen nahezu ungiftig ist. Des Weiteren zeigt Silber ein relativ geringes allergenes Potential. Silber wirkt als antiseptische Substanz in geringen Konzentrationen über einen langen Zeitraum auf eine Vielzahl von Infektionskeimen. Die meisten bekannten Bakterien zeigen darüber hinaus keine Resistenz gegen Silber.

Der Stoff könnte zumindest ein Nebengruppenelement umfassen. Nebengruppenelemente zeichnen sich durch antimikrobielle Wirkung aus. Vor diesem Hintergrund ist denkbar, dass mehrere Nebengruppenelemente gemeinsam vorliegen, um unterschiedlichen Bakterienarten selektiv zu begegnen. Es hat sich in Versuchsreihen gezeigt, dass sich in Bezug auf die antimikrobielle Wirksamkeit eine Rangfolge der verwendeten Stoffe ergibt. Diese lässt sich wie folgt darstellen, Silber ist der wirksamste Stoff, gefolgt von Quecksilber, Kupfer, Cadmium, Chrom, Blei, Kobalt, Gold, Zink, Eisen und schließlich Mangan. Vor diesem Hintergrund ist denkbar, auch Hauptgruppenelemente zu verwenden, welche eine antimikrobielle Wirkung zeigen. Der antimikrobiell wirkende Stoff könnte eine Gold-Silber Mischung umfassen oder ausschließlich aus einer Gold-Silber-Mischung bestehen. Mischungen dieser Art zeigen eine besonders hohe antimikrobielle Wirksamkeit. Es hat sich überraschenderweise gezeigt, dass die Anwesenheit von Gold die antimikrobielle Wirkung von Silber noch steigert.

Zumindest ein Teil der Fasern könnte als Nanofasern ausgestaltet sein. Ein Vliesstoff dieser Ausgestaltung kann besonders leicht und dünn ausgestaltet sein.

Der Vliesstoff könnte eine Reißfestigkeit bei einem spezifischen Flächengewicht von 140 bis 180 g/m² im trockenen Zustand von 0,15 Nlmm² oder mehr und eine Reißdehnung in hydratisiertem Zustand von 150%, bevorzugt von 200% oder mehr aufweisen. Ein solcher Vliesstoff eignet sich besonders gut als Verbandsmaterial, da er problemlos aufgewickelt werden kann.

Der Vliesstoff könnte eine offene Porenstruktur mit einer Luftdurchlässigkeit von 0,5 l/min*cm² aufweisen, wobei dieser Parameter gemäß DIN 9237 ermittelt wird. Ein solcher Vliesstoff eignet sich besonders gut als Verbandsmaterial, da er der Haut erlaubt, Feuchtigkeit abzugeben und zu atmen.

Die austretenden Fasern könnten gerichtet, kontaktlos geführt werden. Eine kontaktlose und definierte Führung der Fasern, bevor diese auf einer Ablageeinrichtung auftreffen, erlaubt eine Modifikation der Fasern. So kann allein die Dauer der Führung bzw. die Richtung der Führung Einfluss auf die Faserlänge, den Faserdurchmesser sowie die Faserstruktur nehmen. Eine gerichtete kontaktlose Führung erzeugt ein homogeneres Faserspektrum, als ein Herstellungsprozess ohne Führung. Ganz konkret kann allein durch die Führung die Breite einer Verteilungskurve sämtlicher Fasereigenschaften eingestellt werden. Hierdurch kann die Menge an Fasern mit unerwünschter Fasergeometrie erheblich reduziert werden.

In einer konstruktiv besonders günstigen Ausgestaltung könnten die Fasern durch eine Absaugeinrichtung geführt werden. Dabei ist denkbar, dass die Fasern durch einen Gasstrom transportiert werden. Sofern der Gasstrom laminar ausgebildet ist, können die Fasern zwischen den laminaren Schichten gestreckt und geformt werden.

Als Gas könnte Luft fungieren. Luft ist ein kostengünstiges Gas, welches sich dadurch auszeichnet, dass ein Herstellungsprozess bei Atmosphärendruck durchgeführt werden könnte. Denkbar ist auch, dass anstelle von Luft weitere Gase, insbesondere Inertgase wie Stickstoff verwendet werden. Hierdurch ist sicher gestellt, dass Faserrohmaterial verarbeitbar ist, welches reaktive Gruppen umfasst oder zu Nachreaktionen nach dem Verlassen des Behältnisses neigt.

Es könnten Fasern hergestellt werden, die einen Durchmesser von 0,3 bis 500 µm aufweisen. Insoweit ist eine Herstellung von Nanofasern möglich, ohne dass ein elektrostatisches Feld verwendet werden muss.

Die Austrittsbereiche des Behältnisses könnten als Durchgänge ausgestaltet sein. Hierbei ist denkbar, dass die Durchgänge kreisförmig, oval oder rechteckförmig ausgestaltet sind. Ganz in Abhängigkeit von der Form der Durchgänge kann auf die Fasergeometrie Einfluss genommen werden.

Die Durchgänge könnten einen Durchmesser oder eine Weite bis 500 µm aufweisen. Diese Dimensionierung hat sich als besonders vorteilhaft für die Erzeugung von Nanofasern und Mikrofasern erwiesen. Die Durchgänge könnten in einem Abstand von einem Zentimeter voneinander positioniert sein. Denkbar ist auch, dass die Durchgänge in mehreren Reihen übereinander angeordnet sind. Hierdurch ist der Durchsatz an Faserrohmaterial auf besonders einfache Weise steigerbar. Durch Verringerung oder Vergrößerung des Durchmessers ist es möglich, Nano- bzw. Mikrofasern im Bereich von 0,3 bis 500 µm zu erzeugen.

Das Behältnis könnte mit bis zu 25.000 Umdrehungen pro Minute rotierbar sein. Durch diese hohe Drehzahl ist es möglich, Nanofasern mit einem Durchmesser von höchstens 100 nm zu erzeugen. Üblicherweise werden Nanofasern durch ein elektrisches Spinnverfahren unter Verwendung eines elektrischen Feldes erzeugt. Durch eine geeignete konstruktive Ausgestaltung des Behältnisses sowie sehr hohe Umdrehungszahlen ist es jedoch möglich, ohne elektrisches Feld Nanofasern zu erzeugen. Durch Wahl der Rotationsgeschwindigkeit und Viskosität des Faserrohmaterials können auch Fasern mit größerem Durchmesser erzeugt werden.

Das Behältnis könnte auf 300° C erwärmbar sein. Die Verwendung von biologisch abbaubaren Stoffen namlich, Gelatine als Faserrohmaterial ermöglicht die Erzeugung von Fasern, die problemlos entsorgbar sind. Des Weiteren können mit diesen Fasern medizinische Produkte wie Wundverbände oder Zellwachstumsträger gefertigt werden.

Dem rotierenden Behältnis könnte eine Ablageeinrichtung zur Aufnahme von Faserrohmaterial zugeordnet sein. Die Ablageeinrichtung könnte ais Plattform ausgestaltet sein, auf der die Fasern zur Bildung eines Faserflors oder Vlieses abgelegt werden können. Denkbar ist auch, dass die Ablageeinrichtung eine rotierende Einrichtung ist, auf welcher Fasern zur Beschichtung eines zylindrischen Körpers oder zur Erzeugung eines Wickelvlieses aufgenommen werden.

Zwischen der Ablageeinrichtung und dem Behältnis könnte eine elektrische Potentialdifferenz bestehen. Diese konkrete Ausgestaltung erlaubt die Fertigung von elektrostatisch aufgeladenen Fasern.

Des Weiteren ist denkbar, dass die elektrische Potentialdifferenz zur Unterstützung der Herstellung von Nanofasern verwendet wird. Hierbei addieren sich die Effekte der Zentripetalkräfte und des elektrischen Feldes, d.h. das Faserrohmaterial wird einerseits durch die Zentripetalkräfte in dünnen Fäden tangential vom rotierenden Behältnis weg geschleudert und des Weiteren durch das elektrische Feld ggf. noch weiter aufgesplittet. Insoweit ist denkbar, einen Fertigungsprozess zu realisieren, durch welchen Fasern im Subnanometerbereich herstellbar sind.

Das Faserrohmaterial könnte bereits in fluidisierter Form in das Behältnis eingebracht werden. Hierdurch ist es möglich, einen kontinuierlichen Prozess durchzuführen, indem nämlich das Faserrohmaterial außerhalb des Behältnisses erwärmt wird. Denkbar ist jedoch auch, das Faserrohmaterial erst zu fluidisieren, nachdem es in das Behältnis eingebracht wurde.

Vliesstoffe der hier beschriebenen Art könnten im medizinischen Bereich Verwendung finden, da sie im Hinblick auf ihre Gewebestruktur und stoffliche Zusammensetzung sehr gut modifizierbar sind. Beispielsweise ist denkbar, die Gewebestruktur so einzustellen, dass sie als Wundverband fungieren können, wenn nämlich die Fasergewebesiruktur mit dem menschlichen Gewebe gut verwachsen kann. Weitere medizinische Anwendungen wie die Verwendung als Zellwachstumsträger sind denkbar.

Der hier beschriebene Vliesstoff eignet sich besonders zur Herstellung eines Wattetupfers oder Tupfers, da er hinreichend stabil ist und desinfizierend wirkt. Weitere Ausrüstungen des Vliesstoffs sind möglich. Der Vliesstoff könnte mit rekombinanten Wachstumsfaktoren, autologen Wachstusmfaktoren, insbesondere thrombozyten-Präparationen, Adhäsionsfaktoren, insbesondere RDG-Peptiden, und/oder autologen Zellpräparationen, insbesondere Knochenmarkaspiraten ausgerüstet sein.

Es gibt nun verschiedene Möglichkeiten, die Lehre der vorliegenden Erfindung in vorteilhafter Weise auszugestalten und weiterzubilden. Dazu ist einerseits auf die nachgeordneten Ansprüche, andererseits auf die nachfolgende Erläuterung bevorzugter Ausführungsbeispiele des erfindungsgemäßen Vliesstoffs anhand der Zeichnung zu verweisen.

In Verbindung mit der Erläuterung der bevorzugten Ausführungsbeispiele anhand der Zeichnung werden auch im Allgemeinen bevorzugte Ausgestaltungen und Weiterbildungen der Lehre erläutert.

### Kurzbeschreibung der Zeichnung

In der Zeichnung zeigen
- Fig. 1: eine REM-Aufnahme eines Vliesstoffs aus Gelatine, in dessen Fasern Silber als antimikrobieller Stoff in Form von nanoskaligen Partikeln verteilt ist,
- Fig. 2: eine REM-Aufnahme des Vliesstoffs aus Fig. 1 in vergrößerter Ansicht,
- Fig. 3: eine REM-Aufnahme einer Faser des Vliesstoffs aus Fig. 1 in vergrößerter Ansicht, welche einen Durchmesser von 4 µm aufweist, und
- Fig. 4: eine REM-Aufnahme eines Faserbündels, welches miteinander verschlungene Fasern aufweist.

### Ausführung der Erfindung

### Ausführungsbeispiel 1:

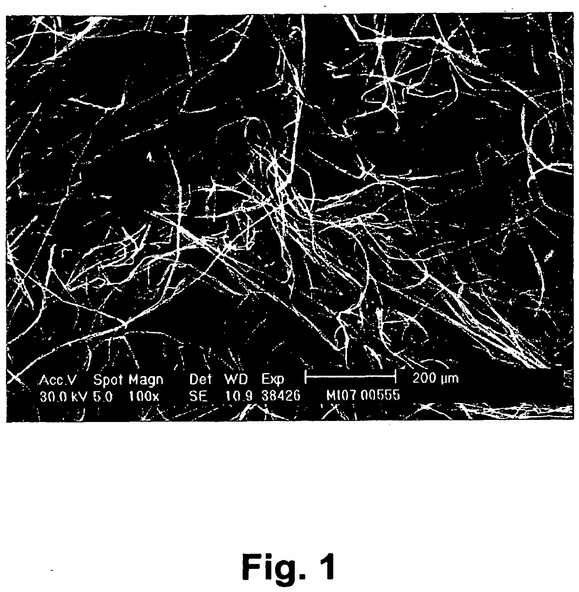

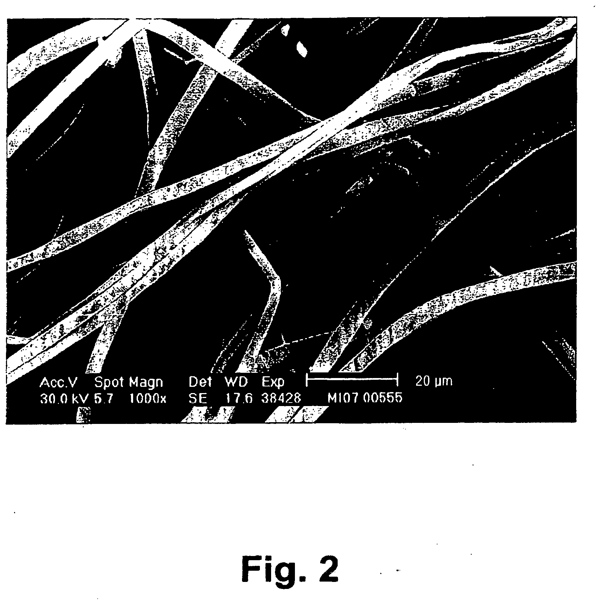

Ein Vliesstoff mit Silber als antimikrobiellem Stoff gemäß den Fig. 1 und 2 wird durch ein Rotationsspinnverfahren wie folgt hergestellt:

Zunächst wird eine 20%ige Gelatine-Lösung hergestellt. Zur Verwendung kommt eine Gelatine des Typs A PIGSKIN der Firma GELITA AG. Die Gelatine wird in Wasser eingerührt. Der Gelatine-Lösung werden 1000 ppm des Feststoffgehalts einer 5%igen wässrigen Silberlösung zugegeben, die Silber in Form von nanoskaligen Partikeln enthält. Es wurde eine Silberlösung der Firma RENT A SCIENTIST, des Typs AGPURE verwendet. Hierdurch entsteht eine Endkonzentration von 50 mg Silber /(kg Gelatine-Lösung).

Darauf verbleibt die Gelatine-Lösung etwa eine Stunde in Ruhe, um aufzuquellen. Anschließend wird die Gelatine-Lösung bei 60°C in einem Ultraschallbad gelöst und darauf ca. 2 Stunden auf einer Temperatur von 80-85°C gehalten. Die Partikel des Silbers können in der Gelatine-Lösung Agglomerate bilden, die durch Rühren der Gelatine-Lösung aufgelöst werden.

Die auf 80-85°C temperierte Gelatine-Lösung wird mittels einer Schlauchpumpe als Faserrohmaterial in das Behältnis einer Vorrichtung zum Rotationsspinnen gemäß der DE 102005048939 A1 geführt.

Das Behältnis hat eine Temperatur von ca. 120°C und rotiert mit einer Drehzahl von 4500 U/min. Im Behältnis befinden sich Ausnehmungen, die als Löcher mit einem Durchmesser von 0,3 mm ausgestaltet sind. Durch die Zentripetalkraft wird das Faserrohmaterial durch die Ausnehmungen gepresst und zu Fasern gesponnen, die durch eine Absaugeinrichtung verstreckt werden. Die Absaugeinrichtung befindet sich unterhalb des Behältnisses.

Nach Vernetzung der Gelatine wird ein antimikrobiell wirksamer, bioresorbierbarer Vliesstoff aus Gelatine, nämlich ein Gelatinevliesstoff, erhalten.

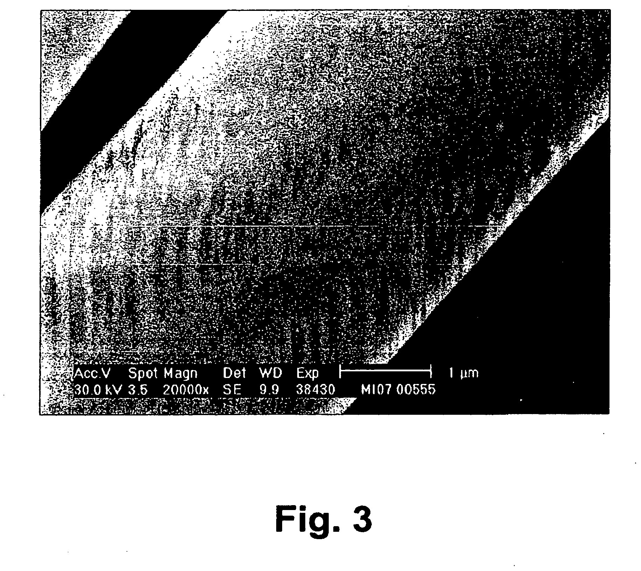

Der Vliesstoff wurde mit einem Rasterelektronenmikroskop (REM) charakterisiert. Die Fig. 1 bis 3 zeigen REM-Aufnahmen des hier beschriebenen Vliesstoffs mit unterschiedlicher Vergrößerung. Gemäß einer Charakterisierung nach ICP (gemäß EN ISO 11885) beträgt die Silberkonzentration im Vliesstoff 44 mg/kg.

In Fig. 2 ist insbesondere im linken unteren Viertel der Figur erkennbar, dass einige Fasern miteinander verschlungen oder verdrillt sind. Das Aufreten dieser Verschlingungen ist charakteristisch für einen Vliesstoff, der durch ein Rotationsspinnverfahren hergestellt wurde.

### Ausführungsbeispiel 2:

Ein Vliesstoff mit Antibiotika wird durch ein Rotationsspinnverfahren wie folgt hergestellt:

Zur Herstellung eines Vliesstoffs wird zunächst eine 20%ige Gelatine-Lösung hergestellt. Zur Verwendung kommt eine Gelatine vom Typ A PIGSKIN gemäß Beispiel 1. Die Gelatine wird in Wasser eingerührt. Diese Gelatine-Lösung verbleibt zum Quellen für eine Stunde in Ruhe. Anschließend wird die Gelatine-Lösung bei 60°C in einem Ultraschallbad gelöst und dann etwa zwei Stunden auf einer Temperatur von 80-85°C gehalten.

Die auf 80-85°C temperierte Gelatine-Lösung wird mittels einer Schlauchpumpe in das Behältnis gemäß der DE 102005048939 A1 geführt. Kurz vor dem Eintritt der Gelatine-Lösung in die Ausnehmungen wird der Gelatine-Lösung eine Ampulle Gentamicin-Lösung (GENTAMICIN 40 der Firma HEXAL AG) beigemengt. Das Behältnis hat eine Temperatur von ca. 120°C und dreht mit einer Drehzahl von 4500 U/min. Durch die Zentripetalkraft wird das Faserrohmaterial aus den am Behältnis befindlichen Ausnehmungen gepresst und zu Fasern gesponnen. Die Fasern werden durch eine Absaugeinrichtung, die sich unterhalb des Behältnisses befindet, verstreckt. Nach Vernetzung der Gelatine wird ein Vliesstoff mit einem eingeschlossenen Antibiotika erhalten, welcher antimikrobiotisch wirkt und zugleich bioresorbierbar ist.

### Ausführungsbeispiel 3 (Vergleichsbeispiel)

Ein Vliesstoff mit nachträglicher Antibiotika-Ausrüstung wird durch ein Rotationsspinnverfahren wie folgt hergestellt:

Zur Herstellung eines Vliesstoffs wird zunächst eine 20%ige Gelatine-Lösung hergestellt. Zur Verwendung kommt eine Gelatine vom Typ A PIGSKIN gemäß Beispiel 1. Die Gelatine wird in Wasser eingerührt. Diese Gelatine-Lösung verbleibt zum Quellen für eine Stunde in Ruhe. Anschließend wird die Gelatine-Lösung bei 60°C in einem Ultraschallbad gelöst und dann etwa zwei Stunden auf einer Temperatur von 80-85°C gehalten.

Die auf 80-85°C temperierte Gelatine-Lösung wird mittels einer Schlauchpumpe in das Behältnis gemäß der DE 102005048939 A1 geführt. Das Behältnis hat eine Temperatur von ca. 120°C und dreht mit einer Drehzahl von 4500 U/min. Durch die Zentripetalkraft wird das Faserrohmaterial aus den am Behältnis befindlichen Ausnehmungen gepresst und zu Fasern gesponnen. Die Fasern werden durch eine Absaugeinrichtung, die sich unterhalb des Behältnisses befindet, verstreckt. Nach Vernetzung der Gelatine wird der Vliesstoff mit einer Lösung von Gentamicin besprüht und danach getrocknet.

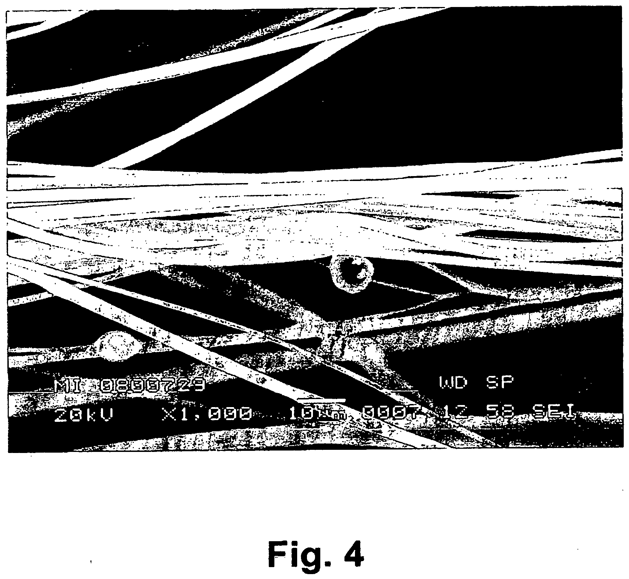

Fig. 4 zeigt einen Vliesstoff, der analog zum Ausführungsbeispiel 1 hergestellt wurde. Bei diesem Vliesstoff sind einige Fasern miteinander verschlungen und bilden ein Faserbündel aus. Durch die Verschlingungen einzelner Fasern werden diese zu einem Faserbündel zusammengefasst und können gegeneinander reversibel verschoben werden. Hierdurch ist es möglich, den Vliesstoff zerstörungsfrei zu dehnen. Bei der Dehnung werden nämlich einzelne Fasern gezogen und relativ zu anderen Fasern verschoben. Durch die Verdrillungen oder Verschlingungen wird sogar begünstigt, dass die Fasern in ihre Position vor der Dehnung zurückkehren. Der Vliesstoff zeigt daher eine hohe Formstabilität.

Hinsichtlich weiterer vorteilhafter Ausgestaltungen und Weiterbildungen der erfindungsgemäßen Lehre wird einerseits auf den allgemeinen Teii der der Beschreibung und andererseits auf die beigefügten Patentansprüche verwiesen. Abschließend sei ganz besonders hervorgehoben, dass die zuvor rein willkürlich ausgewählten Ausführungsbeispiele lediglich zur Erörterung der erfindungsgemäßen Lehre dienen, diese jedoch nicht auf diese Ausführungsbeispiele einschränken.

## Patentansprüche

1. Rotationsspinnverfahren zur Herstellung eines Vliesstoffs, umfassend Fasern aus einem Faserrohmaterial, welches Gelatine aufweist, wobei die Fasern mit einem antimikrobiell wirksamen Stoff und/ oder einem Antibiotikum ausgerüstet sind, wobei das Faserrohmaterial in ein Behältnis gegeben wird, wobei das Behältnis in Rotation versetzt wird und wobei das fluidisierte Faserrohmaterial durch Zentripetalkräfte aus dem Behältnis in Form von Fasern ausgetragen wird, **dadurch gekennzeichnet dass** der antimikrobiell wirksame Stoff und/ oder das Antibiotikum homogen in den Fasern verteilt ist.

2. Rotationsspinnverfahren nach Anspruch 1, **dadurch gekennzeichnet, dass** einige Fasern miteinander verdrillt oder miteinander verschlungen sind oder eine verdrillte Struktur aufweisen.

3. Rotationsspinnverfahren nach Anspruch 1 oder 2, **dadurch gekennzeichnet, dass** einige Fasern miteinander verschlungen sind und ein oder mehrere Faserbündel ausbilden.

4. Rotationsspinnverfahren nach einem der Ansprüche 1 bis 3, **dadurch gekennzeichnet, dass** die Fasern ausschließlich aus Gelatine oder Derivaten der Gelatine gefertigt sind, wobei in und/oder auf den Fasern ein antimikrobieller Stoff und/oder ein Antibiotikum vorliegt.

5. Rotationsspinnverfahren nach einem der Ansprüche 1 bis 4, **dadurch gekennzeichnet, dass** der antimikrobiell wirksame Stoff und/ oder das Antibiotikum in den Fasern nanoskalig vorliegt.

6. Rotationsspinnverfahren nach einem der Ansprüche 1 bis 5, **dadurch gekennzeichnet, dass** der antimikrobiell wirksame Stoff und/ oder das Antibiotikum auf den Fasern verteilt ist.

7. Rotationsspinnverfahren nach einem der Ansprüche 1 bis 6, **dadurch gekennzeichnet, dass** der antimikrobiell wirksame Stoff Silber enthält.

8. Rotationsspinnverfahren nach einem der Ansprüche 1 bis 7, **dadurch gekennzeichnet, dass** zumindest ein Teil der Fasern als Nanofasern ausgestaltet ist.

9. Rotationsspinnverfahren nach einem der Ansprüche 1 bis 8, wobei ein Vliesstoff hergestellt wird, der durch eine offene Porenstruktur mit einer Luftdurchlässigkeit von 0,5 l/min*cm² gekennzeichnet ist.

10. Rotationsspinnverfahren nach einem der Ansprüche 1 bis 19, **dadurch gekennzeichnet, dass** die austretenden Fasern gerichtet, kontaktlos geführt werden.

11. Rotationsspinnverfahren nach einem der Ansprüche 1 bis 10, **dadurch gekennzeichnet, dass** die austretenden Fasern durch eine Absaugeinrichtung geführt werden.

12. Rotationsspinnverfahren nach einem der Ansprüche 1 bis 11, **dadurch gekennzeichnet, dass** Fasern hergestellt werden, die einen Durchmesser von 0,3 bis 500 µm aufweisen.

13. Rotationsspinnverfahren nach einem der Ansprüche 1 bis 12, **dadurch gekennzeichnet, dass** die Austrittsbereiche des Behältnisses als Durchgänge mit einem Durchmesser bis 500 µm ausgestaltet sind.

14. Rotationsspinnverfahren nach einem der Ansprüche 1 bis 13, **dadurch gekennzeichnet, dass** dass das Behältnis mit bis zu 25 000 Umdrehungen pro Minute rotierbar ist.

15. Rotationsspinnverfahren nach einem der Ansprüche 1 bis 14 **dadurch gekennzeichnet, dass** dass das Behältnis auf 300°C erwärmbar ist.

16. Rotationsspinnverfahren nach einem der Ansprüche 1 bis 15, **dadurch gekennzeichnet, dass** die Fasern auf einer Ablageeinrichtung abgelegt werden, wobei zwischen der Ablageeinrichtung und dem Behältnis eine elektrische Potentialdifferenz besteht.

17. Verwendung eines Vliesstoffs hergestellt nach einem Verfahren der Ansprüche 1 bis 16 zur Herstellung eines Arzneimittels für die Wundheilung.

18. Verwendung eines Vliesstoffs hergestellt nach einem Verfahren der Ansprüche 1 bis 16 zur Herstellung eines Wattetupfers.

## Claims

1. Rotational spinning method for the production of a nonwoven fabric comprising fibers of a fiber raw material which comprises gelatin, wherein the fibers are produced exclusively from gelatin or derivatives of gelatin, the fibers having been provided with an antimicrobially effective substance and/or an antibiotic, the fiber raw material being introduced into a container, the container being caused to rotate and the fluidized fiber raw material being discharged by centripetal forces from the container in the form of fibers, **characterized in that** the antimicrobially effective substance and/or the antibiotic is homogeneously distributed in the fibers.

2. Rotational spinning method according to Claim 1, **characterized in that** some fibers are twisted with one another or interlaced with one another or have a twisted structure.

3. Rotational spinning method according to Claim 1 or 2, **characterized in that** some fibers are interlaced with one another and form one or more fiber bundles.

4. Rotational spinning method according to any of Claims 1 to 3, **characterized in that** the antimicrobially effective substance and/or the antibiotic is present in the fibers at the nanoscale level.

5. Rotational spinning method according to any of Claims 1 to 4, **characterized in that** the antimicrobially effective substance and/or the antibiotic is distributed on the fibers.

6. Rotational spinning method according to any of Claims 1 to 5, **characterized in that** the antimicrobially effective substance contains silver.

7. Rotational spinning method according to any of Claims 1 to 6, **characterized in that** at least a part of the fibers is in the form of nanofibers.

8. Rotational spinning method according to any of Claims 1 to 7, a nonwoven fabric being produced which is **characterized by** an open pore structure having an air permeability of 0.5 l/min*cm².

9. Rotational spinning method according to any of Claims 1 to 8, **characterized in that** the emerging fibers are guided in a directed and noncontact manner.

10. Rotational spinning method according to any of Claims 1 to 9, **characterized in that** the emerging fibers are guided by a suction device.

11. Rotational spinning method according to any of Claims 1 to 10, **characterized in that** fibers which have a diameter of from 0.3 to 500 µm are produced.

12. Rotational spinning method according to any of Claims 1 to 11, **characterized in that** the exit regions of the container are in the form of passages having a diameter of up to 500 µm.

13. Rotational spinning method according to any of Claims 1 to 12, **characterized in that** the container is rotatable at up to 25 000 revolutions per minute.

14. Rotational spinning method according to any of Claims 1 to 13, **characterized in that** the container can be heated to 300°C.

15. Rotational spinning method according to any of Claims 1 to 14, **characterized in that** the fibers are laid on a laying device, an electrical potential difference existing between the laying device and the container.

16. Use of a nonwoven fabric produced according to a method of Claims 1 to 15 for the production of a drug for wound healing.

17. Use of a nonwoven fabric produced according to a method of Claims 1 to 15 for the production of a cotton swab.

## Revendications

1. Procédé de filage par rotation pour la fabrication d'un non-tissé, comprenant des fibres d'un matériau brut fibreux, qui comprend de la gélatine, les fibres sont fabriquées exclusivement à partir de gélatine ou de dérivés de gélatine, les fibres étant munies d'une substance à action antimicrobienne et/ou d'un antibiotique, le matériau brut fibreux étant introduit dans un récipient, le récipient étant mis en rotation et le matériau brut fibreux fluidisé étant déchargé du récipient sous la forme de fibres par les forces centripètes, **caractérisé en ce que** la substance à action antimicrobienne et/ou l'antibiotique est réparti de manière homogène dans les fibres.

2. Procédé de filage par rotation selon la revendication 1, **caractérisé en ce que** quelques fibres sont torsadées les unes avec les autres ou sont entrelacées les unes avec les autres ou présentent une structure torsadée.

3. Procédé de filage par rotation selon la revendication 1 ou 2, **caractérisé en ce que** quelques fibres sont entrelacées les unes avec les autres et forment un ou plusieurs faisceaux de fibres.

4. Procédé de filage par rotation selon l'une quelconque des revendications 1 à 3, **caractérisé en ce que** la substance à action antimicrobienne et/ou l'antibiotique est présent à l'échelle nanométrique dans les fibres.

5. Procédé de filage par rotation selon l'une quelconque des revendications 1 à 4, **caractérisé en ce que** la substance à action antimicrobienne et/ou l'antibiotique est réparti sur les fibres.

6. Procédé de filage par rotation selon l'une quelconque des revendications 1 à 5, **caractérisé en ce que** la substance à action antimicrobienne contient de l'argent.

7. Procédé de filage par rotation selon l'une quelconque des revendications 1 à 6, **caractérisé en ce qu'**au moins une partie des fibres est conçue sous la forme de nanofibres.

8. Procédé de filage par rotation selon l'une quelconque des revendications 1 à 17, un non-tissé étant fabriqué, qui est **caractérisé par** une structure poreuse ouverte ayant une perméabilité à l'air de 0,5 l/min*cm².

9. Procédé de filage par rotation selon l'une quelconque des revendications 1 à 8, **caractérisé en ce que** les fibres sortantes sont dirigées de manière orientée, sans contact.

10. Procédé de filage par rotation selon l'une quelconque des revendications 1 à 9, **caractérisé en ce que** les fibres sortantes sont dirigées par un dispositif d'aspiration.

11. Procédé de filage par rotation selon l'une quelconque des revendications 1 à 10, **caractérisé en ce que** des fibres présentant un diamètre de 0,3 à 500 µm sont fabriquées.

12. Procédé de filage par rotation selon l'une quelconque des revendications 1 à 11, **caractérisé en ce que** les zones de sortie du récipient sont conçues sous la forme de passages d'un diamètre allant jusqu'à 500 µm.

13. Procédé de filage par rotation selon l'une quelconque des revendications 1 à 12, **caractérisé en ce que** le récipient peut tourner à une vitesse pouvant atteindre 25 000 tours par minute.

14. Procédé de filage par rotation selon l'une quelconque des revendications 1 à 13, **caractérisé en ce que** le récipient peut être porté à une température de 300 °C.

15. Procédé de filage par rotation selon l'une quelconque des revendications 1 à 14, **caractérisé en ce que** les fibres sont déposées sur un dispositif de dépôt, une différence de potentiel électrique existant entre le dispositif de dépôt et le récipient.

16. Utilisation d'un non-tissé fabriqué par un procédé selon les revendications 1 à 15 pour la fabrication d'un médicament pour la guérison des blessures.

17. Utilisation d'un non-tissé fabriqué par un procédé selon les revendications 1 à 15 pour la fabrication d'une compresse d'ouate.
